# EUROPEAN PATENT SPECIFICATION

(11) **EP 2 916 775 B1**
(45) Date of publication and mention of the grant of the patent: **07.06.2017**
(21) Application number: 13802531.7
(22) Date of filing: 12.11.2013
(51) Int. Cl.: A61F 2/36

(54) **TEMPORARY PROSTHESIS HEAD**
KOPF EINER PROVISORISCHEN PROTHESE
TÊTE DE PROTHÈSE PROVISOIRE

(30) Priority: 12.11.2012 LU 92093
(43) Date of publication of application: 16.09.2015
(73) Proprietor: Université du Luxembourg, 1511 Luxembourg-Limpertsberg (LU); Kelm, Jens, 66440 Blieskastel (DE)
(72) Inventor: KELM, Jens, 66440 Blieskastel (DE); MAAS, Stefan, 54450 Freudenburg (DE); COLLÉ, Claude, 5552 Remich (LU)
(74) Representative: Dennemeyer & Associates S.A.
(86) International application number: PCT/EP2013/003403
(87) International publication number: WO 2014/072076

(56) References cited:
- WO-A1-2010/015877
- WO-A2-2009/073781
- DE-A1- 10 156 610
- DE-U1-202009 012 964
- US-A1- 2009 157 189
- US-A1- 2009 175 978

## Description

### Technical field

The present invention relates to device for manufacturing a prosthesis head, and more particularly a temporary prosthesis head for a hip implant. Moreover the present invention relates to a method of manufacturing a prosthesis head, and more particularly to a method of manufacturing a temporary prosthesis head for a hip implant.

### Background of the invention

In conventional hip joint replacement the femoral head is replaced by a femoral implant with an artificial implant head. In the same operation, the acetabulum, i.e. the hip socket in the pelvic bone, is excavated and receives an acetabular cup adapted for engaging with the head of the femoral implant. Although this is increasingly becoming a standard procedure, with about 300,000 cases per year in the U.S. alone, it is estimated that about 1.5 per cent of the patients suffer from an infection following this surgery. The conventional treatment for these infections foresees removing the implanted acetabular cup from the acetabulum as well as removing the femoral implant from the femur. The femoral implant is entirely replaced by a temporary implant, known in the art as a "spacer", during the recovery period. Once the infection has healed, the acetabular cup is re-implanted in the pelvic bone, and the spacer is again removed from the femur and replaced by the permanent femoral implant.

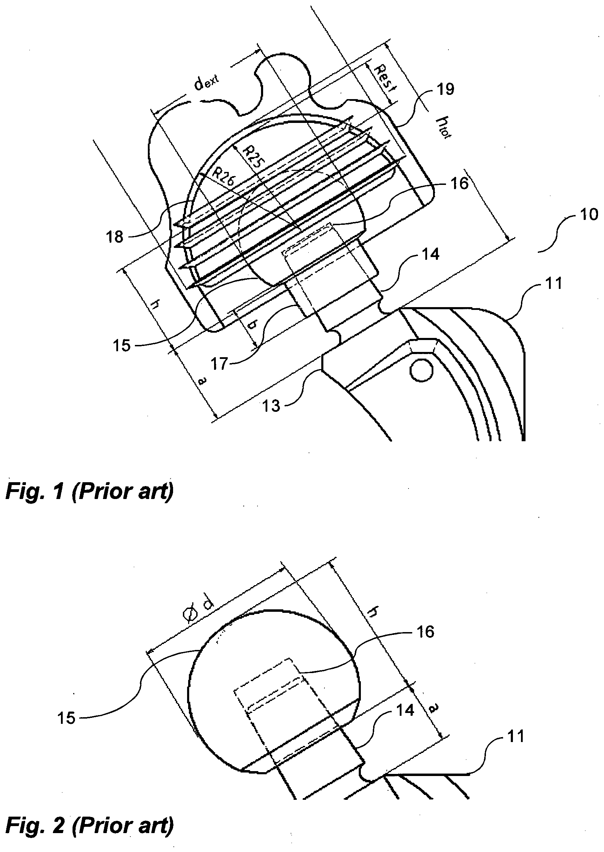

Fig. 1 shows a cross section of the typical post hip replacement situation. As is known in the art, a femoral prosthesis 10 generally comprises a femoral implant body 11 with a distal end embedded in the femur and a proximal end 13 located adjacent the patient's hip joint. An implant neck 14 protrudes in the proximal direction from the proximal end 13 of the femoral implant body 11. The implant neck 14 may have a tapered proximal end, such as a Morse taper, as is known in the art, to accommodate a substantially spherical prosthesis head 15. The prosthesis head 15 is provided with a matching tapered socket 16 and can be pushed onto the implant neck 14. The prosthesis head 15 and the implant neck 14 are then retained by friction. The prosthesis head 15 typically takes the form of a joint ball or bearing, sometimes with a cylindrical protrusion in the form of a hub 17 extending in the distal direction, surrounding the tapered socket 16 for receiving and engaging with the implant neck 14. The prosthesis head 15 rests against an acetabular cup 18 which has been implanted into the pelvic bone 19. In general, such an acetabular cup 18 comprises an inlay, typically made from polyethylene (PE), ceramics, or other suitable inert materials known in the art for this purpose. The acetabular cup 18 essentially has the shape of a concave, or hollow, half-sphere. The external diameter of the spherical prosthesis head 15 is adapted to and essentially corresponds to the inner diameter of the inlay of the acetabular cup 18. It is further known to provide the acetabular cup with an inlay that is usually selected from polyethylene (PE), porcelain, or ceramics. Together, the prosthesis head 15 and the acetabular cup 18 form the new hip joint.

In an alternative embodiment, it is known to provide the acetabular cup 18 in a conical form.

When a complication, such as an infection of the pelvic bone, occurs, the typical treatment foresees the temporary removal of the implanted acetabular cup 18 as well as the entire femoral implant body 11. A temporary implant, known as "spacer" in the art, is then embedded in the femur. It is noted that, during the healing period, no acetabular cup 18 is present, and the head of the spacer or of the temporary implant rests in direct contact with the pelvic bone. The head of the spacer is therefore dimensioned to fill the cavity vacated by removing the implanted acetabular cup from the pelvic bone. Frequently the spacer body is made from biocompatible material, such as poly methyl methacrylate (PMMA), and it may further comprise one or more pharmaceutically active substances, such as an antibiotic or antimycotic, which are released from the spacer material over time.

Fig. 2 shows an alternative form of a prosthesis head 15 that is also known in the art. In contrast with the illustration of Fig. 1, the prosthesis head 15 is without a cylindrical hub extending in the distal direction. An implant neck 14 protrudes in the proximal direction from the femoral implant body 11. In the absence of a cylindrical hub, it is the tapered socket 16 provided inside the prosthesis head 15 which receives and engages with the implant neck 14. The tapered socket 16 can be pushed onto the matching implant neck 14. The prosthesis head 15 and the implant neck 14 are then retained by friction. The prosthesis head 15 typically takes the form of a joint ball or bearing.

However, the removal of the implant from the femur places this bone at increased risk of mechanical or other damage, which may be permanent. Moreover, being made from PMMA or other similar materials, the known temporary spacer suffers from a lack of mechanical strength which may result in dislocation, loosening and, in the extreme, breakages. In particular a breakage necessitates a surgical removal of the spacer, which is a severe complication that further inconveniences the patients.

Consequently, there is a need to address the deficiencies that exist in the current state of the art with regard to conventional approaches. Such deficiencies and other needs are addressed by one or more of the various embodiments of the present invention. It is therefore an object of the present invention to overcome or at least ameliorate one or more of the deficiencies that are identified hereinabove. It is further an object of certain embodiments of the present invention to increase the mechanical strength of the implant whilst at the same time allowing to treat the infected hip. It is another object of certain embodiments of the present invention to reduce the inconvenience to the patient resulting from follow-up surgery following infection.

The closest prior art is disclosed in WO 2009/073781 which discloses a device for manufacturing a prosthesis head according to the preamble of claim 1, and in US 2009/175978 which discloses a method according to the preamble of claim 6.

### Summary of the invention

The present invention is based on the realisation that it is sometimes not possible or advisable to remove the entire implant. In accordance with the present invention it is therefore proposed to remove the acetabular cup from the infected site, to leave the femoral implant in place within the femur and to merely replace the implant head by a temporary prosthesis head, or spacer head.

According the present invention there is provided a device for manufacturing a prosthesis head. The device comprises a mould and a core positioning element. The mould has a substantially spherical cavity, optionally in the form of a substantially spherical segment. A core positioning means has a longitudinal axis and comprises a first end. The first end is adapted for receiving a socket of a core element of a prosthesis head. The mould comprises means for receiving the core positioning means, so that the first end of the core positioning means rests within the substantially spherical mould cavity. The means for receiving the core positioning means further align the longitudinal axis of the core positioning means with a radial direction with respect to the spherical cavity.

The core positioning means may further comprise means for adjusting in the longitudinal direction the position of the first end of the core positioning means. This allows the centre point of a core element received on the first end of the core positioning means to be displaced in the radial direction with respect to the centre point of the spherical mould cavity.

The mould comprises a first mould portion and a second mould portion. The first mould portion may comprise a first substantially semi-spherical concave recess, optionally in the form of a substantially semi-spherical segment. The second mould portion may comprise a second substantially semi-spherical concave recess, optionally in the form of a substantially semi-spherical segment. The first mould portion and the second mould portion may be adapted for being placed in abutment with each other when in use, so that the semi-spherical concave recesses face each other and are aligned and placed in tight abutment, thereby forming an essentially spherical cavity for manufacturing a prosthesis head.

In the first mould portion the means for aligning the longitudinal axis of the core positioning means comprise, according to the invention, a first substantially semi-cylindrical channel extending from the first substantially semi-spherical concave recess to an adjacent side wall.

In the second mould portion the means for aligning the longitudinal axis of the core positioning means comprise, according to the invention, a second substantially semi-cylindrical channel extending from the second substantially semi-spherical concave recess to an adjacent side wall.

The core positioning means may be of substantially the same diameter as the essentially semi-cylindrical concave recesses of the first and second mould portions.

The core positioning means may comprise a neck portion at its proximal end for attaching a core element.

The core positioning means may further comprise a bolt, wherein the neck portion is provided at the proximal end of the bolt.

The core positioning means may further comprise means for adjusting the position of its neck portion to a predetermined position when placed into the substantially spherical cavity.

The means for adjusting the position of the neck portion to a predetermined position may comprise an outer threading at the distal end of the bolt.

According to another aspect of the present invention there is provided a method of manufacturing a prosthesis head, comprising the steps of
a) providing a core element;
b) providing a core positioning means having a longitudinal axis and comprising a first end, the first end adapted for receiving a socket of a core element of a prosthesis head;
c) providing a mould, the mould comprising a substantially spherical cavity, wherein the mould further comprises means for receiving the core positioning means;
d) attaching the core element to the first core positioning means end;
e) introducing the core positioning means into the mould, so that the first end of the core positioning means rests within the spherical mould cavity and the longitudinal axis of the core positioning means is aligned with a radial direction with respect to the substantially spherical cavity; and
f) filling the remainder of the substantially spherical cavity with a coating material for coating the core element.

The step c) further comprises
providing a first mould portion, the first mould portion comprising a first semi-spherical concave recess and a first semi-cylindrical channel extending from the first semi-spherical concave recess of the mould along the intersecting plane to a first proximal side wall; and
providing a second mould portion, the second mould portion comprising a second semi-spherical concave recess and a second semi-cylindrical channel extending from the second semi-spherical concave recess of the mould along the intersecting plane to a second proximal side wall.

The step b) may further comprise providing a core positioning means of substantially the same diameter as the first and second semi-cylindrical concave recesses of the first and second mould portions, the core positioning means comprising a neck portion for attaching a core element at the first end of the core positioning means.

The step d) may further comprise attaching the core element to the neck portion of the first end of core positioning means.

The step f) may further comprise the steps of:
f1) providing a mouldable coating material;
f2) inserting the mouldable coating material into the first and second semi-spherical concave recesses of the first and second mould portions;
f3) inserting the core element into the mouldable material provided in the first semi-spherical concave recess of the first mould portion, such that the attached core positioning means is inserted into the first semi-cylindrical channel;
f4) placing the second mould portion in abutment with the first mould portion so that the semi-spherical concave recesses face each other and are aligned and placed in tight abutment such that the semi-cylindrical concave channels are also aligned with each other, whereby the core element is inserted into the mouldable material provided in the second semi-spherical concave recess of the second mould portion,
f5) allowing the mouldable coating material to cure;
f6) separating the first mould portion from the second mould portion, and removing the core positioning means from the coated core element, such that a substantially spherical prosthesis head is obtained in the form of core element coated with a moulded outer coating.

The axial position of the core element in the mould may be adjusted such that a desired axial displacement between the core element and the outer coating results.
The length of the core positioning means may be selected such that a desired axial displacement between the core element and the outer coating results.

The method may further comprise adjusting the length of the core positioning means.
The method may further comprise selecting a core positioning means of desired length.
The mouldable coating material may be selected from PMMA.
The mouldable coating material may comprises at least one pharmaceutically active component, optionally in the form of an antibiotic and/or antimycotic.
The core element may be selected from titanium.

Other objects, features, and advantages of the present invention will become more apparent from the following detailed description when read in conjunction with the accompanying drawings.

### Brief description of the drawings

These aspects and further embodiments of the present invention will now be described with reference to the following non-limiting Figures.
Fig 1 illustrates schematically a known implant body and prosthesis head.
Fig 2 illustrates schematically a second example of a known implant body and prosthesis head.
Fig 3 is a drawing of a first embodiment of a prosthesis head in accordance with the present invention.
Fig 4 and Fig 5 show a detailed cross section of a second embodiment of a prosthesis head in accordance with the present invention.
Fig 6 is an isometric view of a first mould portion in accordance with the present invention.
Fig 7 is an isometric view of a second mould portion in accordance with the present invention.
Figs 8A and 8B illustrate a cross-section and a top view of a core positioning means in accordance with the present invention, when placed into a first mould portion.
Fig 9 is an isometric view of a core positioning means in accordance with the present invention, when placed into a first mould portion.
Fig 10 is an isometric view of a bolt in accordance with the present invention.
Fig 11 is an isometric view of a sleeve in accordance with the present invention.
Fig 12 is an isometric view of a holding plate in accordance with the present invention.
Figs 13 a) to e) are illustrations of the placement of the core element in the outer coating for different sizes of prosthesis heads in accordance with the present invention.

### Detailed technical description of the invention

The present invention relates to mould for manufacturing a prosthesis head, and more particularly to a temporary prosthesis head for a hip implant. Moreover the present invention relates to a method of manufacturing a prosthesis head, more particularly a temporary prosthesis head for a hip implant. Particular embodiments will be described in the following with reference to the non-limiting drawings.

The present invention is based on the concept that it is sometimes not possible or advisable to remove the entire implant. The inventors therefore proposed to leave the femoral implant in place in the femur and to merely remove the acetabular cup and replace the femoral implant head by a suitable prosthesis head in accordance with the present invention.

### The prosthesis head

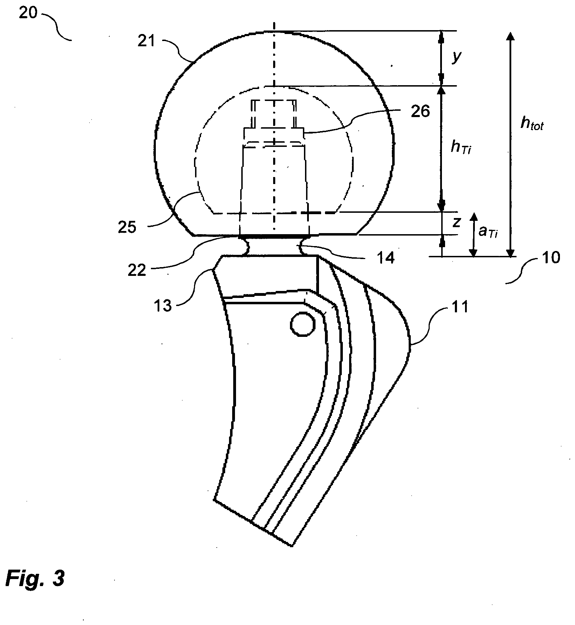

A first example of a prosthesis head resulting from the present invention is illustrated in Fig 3. A femoral prosthesis 10 generally comprises a femoral implant body 11 with a distal end embedded in the femur (not shown for simplicity) and a proximal end 13 located adjacent the patient's hip joint. An implant neck 14 protrudes in the proximal direction from the proximal end 13 of the femoral implant body 11. The implant neck 14 may have a tapered proximal end, such as a Morse taper, as is known in the art.

In accordance with a first aspect a prosthesis head 20 for hip implants comprises a core element 25, which is surrounded by an outer coating 21. The core element 25 further comprises a socket 26, so that it is adapted for receiving a neck of a femoral implant body. It may be advantageous to provide the socket 26 in the form of a blind bore.

The socket 26 may preferably have a conical or tapered shape, so that is adapted for receiving a conical or tapered neck of a femoral implant body.

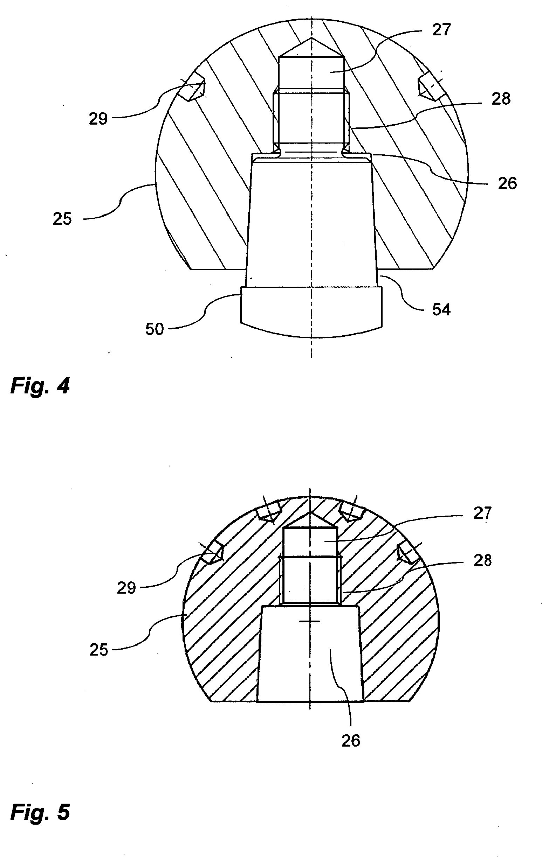

The outer coating 21 of the prosthesis head 20 is of essentially spherical shape. Advantageously the outer coating 21 may have an essentially circular cut-out 22, which may be oriented to substantially coincide with the socket 26 in the core element 25.
In accordance with the geometry of the patient's anatomy, the core element 25 and the coating 21 may be displaced by a predetermined distance along the axis of the socket 26. The calculation of this displacement is described in more detail below.
Inside the socket 26 the core element 25 may further comprise a bore 27 of smaller diameter, as shown in Fig 4. Preferably the bore 27 may be provided in the form of a blind bore. Moreover, the bore 27 may be provided with a threading 28. The threading 28 allows the core element 25 to be fixed to a neck 54 of a core positioning means 50 during manufacturing of the prosthesis head 20.
The core element 25 may preferably be selected from metal, optionally from titanium (Ti). It may further be preferred that the core element 25 is essentially spherical, and more advantageously in the form of a spherical section.
The outer surface of the core element 25 may further comprise a plurality of recesses 29. These recesses 29 may be provided in the form of a number of blind bores which may be distributed evenly or unevenly around the circumference of the core element 25, as illustrated in Fig 5. The recesses 29 allow the outer coating 21 to engage and interlock with the outer surface of the core element 25 to form a mechanically stable prosthesis head 20 during the manufacturing, respectively moulding process.

The outer coating 21 of the prosthesis head 20 may be selected from plastics material, preferably from poly (methyl methacrylate) (PMMA). The plastics material selected for outer coating 21 may optionally comprise at least one pharmacologically active agent, preferably in the form of one of antibiotic or antimycotic. It will usually be preferred to provide the outer coating 21 in essentially spherical shape, and more advantageously in the form of a spherical section.
The diameter of the prosthesis head 20 is advantageously selected to essentially correspond to the diameter of the acetabular cavity after the acetabular cup has been removed from the infected site.

Referring again to the particular example shown in Fig 3, a prosthestic head, or spacer, 20 for hip implants comprises a substantially spherical core element 25 which is surrounded by an outer coating 21. The core element 25 further comprises a socket 26, so that it is adapted for receiving a neck of a femoral implant body. The socket 26 is in the form of a blind bore with a conical or tapered shape, so that is adapted for receiving a conical, or tapered neck of a femoral implant body. The outer coating 21 of the prosthesis head 20 has a cut-out 22, which substantially coincides with the socket 26 in the core element 25. Inside the socket 26 the core element 25 further comprises a bore 27 of smaller diameter. The bore 27 has the form of a blind hole and is provided with an internal threading 28. The internal threading 28 allows fixing the core element 25 to a neck of a core positioning means during manufacturing of the prosthesis head 20. The core element 25 is selected from titanium (Ti). Moreover the core element 25 is essentially spherical. The outer surface of the core element 25 further comprises a plurality of recesses 29. These recesses 29 are provided in the form of a number of blind bores which may for instance be distributed in two circles around the circumference of the core element 25. The recesses 29 allow the outer coating 21 to engage and interlock with the outer surface of the core element 25 to form a mechanically stable prosthesis head 20. The outer coating 21 of the prosthesis head 20 is selected from poly (methyl methacrylate) (PMMA). The plastics material selected for outer coating 21 may optionally comprise at least one pharmacologically active agent, preferably in the form of one of antibiotic or antimycotic. Furthermore, the outer coating 21 has an essentially spherical shape.

### Determining the dimensions of the prosthesis heads

Referring to Fig 1 and Fig 2, the following dimensions have been obtained from conventional prosthesis implants:

**Table 1: dimensions of conventional implants**

| | S | M | L₂₈ | L₃₂ | XL | XXL |
|---|---|---|---|---|---|---|
| *a* + *h* [mm] | 30.1 | 30.8 | 33.6 | 35.6 | 40 | 40.2 |
| *dₑₓₜ* [mm] | 28 | 28 | 28 | 32 | 32 | 28 |
| *Rest* [mm] | 11 | 11 | 11 | 9 | 9 | 11 |
| *x* [mm] | 1 | 1 | 1 | 1 | 1 | 1 |
| *hₜₒₜ* [mm] | 42.1 | 42.8 | 45.6 | 45.6 | 50 | 52.2 |

The total height *hₜₒₜ* can be calculated from the measured values listed in Table 1 for the six different original sizes (labelled from "S" to "XXXL") of the prostheses, wherein:
- *a* = distance border of ball to prosthesis shoulder
- *h* = height of the respective ball 15
- *Rest = r_{socket} -dₑₓₜ*/2 (ball radius)
- *x* = distance between socket joint and pelvic bone (approximately 1 mm)
- *hₜₒₜ* = (*a* + *h)* + *Rest* + *x* = height to be reached with the spacer 20 of the present invention

Since it results that the total heights *hₜₒₜ* for the sizes L₂₈ und L₃₂ are the same, these have been allocated one standard size L, wherein the index 28, respectively 32, denotes the respective ball diameters in millimetres for the original femoral prosthesis 10. The index is not relevant for the present invention and will be ignored in the following.
The core element 25, preferably in the form of a titanium ball, is arranged at a predetermined position on the conical part of the implant neck 14. This neck geometry is fixed and cannot be varied. Accordingly, the only variation in geometry can be achieved by adjusting the internal configuration of the prosthesis head, wherein the spherical outer coating 21 is displaced along the axial direction with respect to the core element 25.
The total height h*ₜₒₜ*" (cf. Fig. 1 and Table 1) of the prosthesic head 20 is axially adjustable via the dimensions *y* and *z*, wherein the sum of *y* + *z* remains constant for each size of the prosthesis head 20. The dimensions *y* and *z* result from the axial placement of the core element 25 in the outer coating 21. Likewise constant and due to construction are the dimensions a and *h* of the core element 25, respectively the implant neck 14 (see Figs. 1 and 2). For the purpose of clearer distinction the index "Ti" has been introduced for these two dimensions, *a_{Ti}* and *h_{Ti}*, in order to indicate that these relate to the new spacer construction in accordance with the present invention.

**Table 2: dimensions of the prosthesis heads in accordance with the present invention, so that for each size "hₜₒₜ" remains unchanged with respect to the corresponding dimensions given in Table 1**

| | S | M | L₂₈ | L₃₂ | XL | XXL |
|---|---|---|---|---|---|---|
| *a_{Ti}* [mm] | 12.9 | 12.9 | 12.9 | 12.9 | 12.9 | 12.9 |
| *h_{Ti}* [mm] | 27 | 27 | 27 | 27 | 27 | 27 |
| *hₜₒₜ* [mm] | 42.1 | 42.8 | 45.6 | 45.6 | 50 | 52.2 |
| *y* [mm] | 2.2 | 2.9 | 5.7 | 5.7 | 10.1 | 12.3 |
| *z* [mm] | 12.1 | 11.4 | 8.6 | 8.6 | 4.2 | 2 |

The dimensions to be used for the prosthesis head 20 in accordance with the present invention can then be calculated as:
- *y* = h*ₜₒₜ* - (a_{Ti} + h_{Ti})
- *z* = height of the mould (e.g. 41,3mm) - (*y* + *h_{Ti}*)

### The mould

In accordance with an aspect of the present invention, a device is proposed that allows the manufacturing of a prosthesis head.

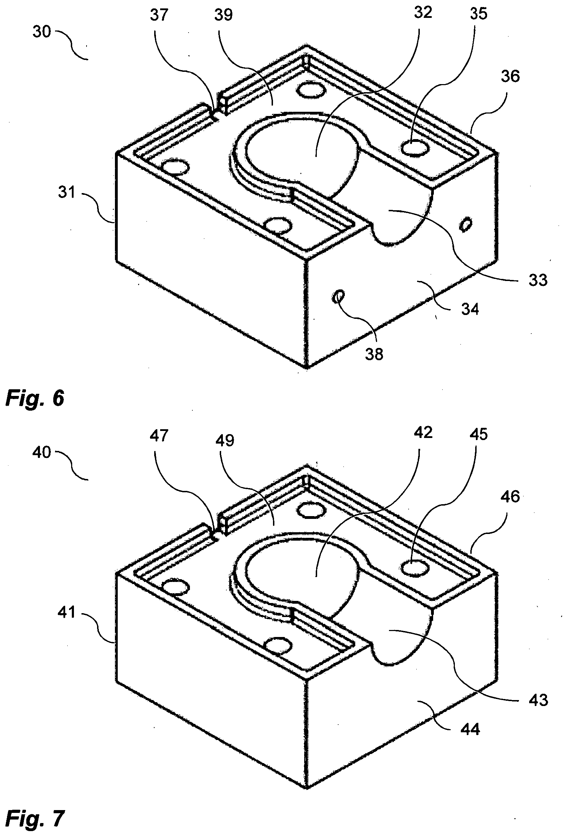

Fig 6 shows a first portion of the manufacturing device in the form of a first mould portion 30. The first mould portion 30 is made from a substantially die-shaped block which comprises four sidewalls, a first face 31 and a first rear face. The first face 31 is arranged so that, when in use, it faces the second face of a second mould portion (described below with reference to Fig 7). Advantageously, the block may be made from polytetrafluoroethylene (PTFE), also known as Teflon^{®}. Of course, it is understood that other suitable materials might also be used.

As further illustrated in Fig 6, a substantially semi-spherical recess 32 is formed in the centre of the first face 31 of the first mould portion 30. Moreover, a semi-cylindrical recess 33 extends along the first face 31 towards an adjacent sidewall 34. Four holes 35 are foreseen, extending vertically through the first face 31 and through the first mould portion 30. The holes 35 are adapted for receiving fitting studs or bolts.

Moreover, a rim 36 is provided along the length of the edges of the first face 31 as well as along the edge of the intersection of the semi-spherical recess 32 and the semi-cylindrical recess 33 with the first face 31, respectively. The rim 36 circumscribes a recessed plane 39 in the first face 31. In addition, a groove 37 is provided in a central section of the rim 36, preferably diametrically opposite the semi-cylindrical recess 33. Whilst the rim 36 serves to ensure tight abutment with the other mould portion during the moulding process, the recessed plane 39 allows to collect excess coating material when, in use, the mould portions are tightly pressed together.

Fig 7 shows a second portion of the manufacturing device in the form of a second mould portion 40. Like the first mould portion 30, the second mould portion 40 is made from a substantially die-shaped block which comprises four sidewalls, a second face 41 and a second rear face. The second face 41 is arranged so that, when in use, it faces the first face 31 of the first mould portion 30 (described above with reference to Fig 6). Advantageously, the block may be made from polytetrafluoroethylene (PTFE), also known as Teflon®. Of course, it is understood that other suitable materials might also be used.

As further illustrated in Fig 7, a substantially semi-spherical recess 42 is formed in the centre of the second face 41 of the second mould portion 40. Moreover, a semi-cylindrical recess 43 extends along the second face 41 towards an adjacent sidewall 44. Four holes 45 are foreseen, extending vertically through the first face 41 and through the first mould portion 40. The holes 45 are adapted for receiving fitting studs or bolts.

Moreover, a rim 46 is provided along the length of the edges of the first face 41 as well as along the edge of the intersection of the semi-spherical recess 42 and the semi-cylindrical recess 43 with the second face 41, respectively. In addition, a groove 47 is provided in a central section of the rim 46, preferably diametrically opposite the semi-cylindrical recess 43. The rim 46 further circumscribes a recessed plane 49 in the first face 41. Whilst the rim 46 serves to ensure tight abutment with the other mould portion, the recess allows to collect excess coating material when, in use, the mould portions are tightly pressed together.

Both the first mould portion 30 and the second mould portion 40 each comprise semi-spherical concave recesses 32, 42 in their central sections. In use, these semi-spherical concave recesses are aligned and placed in tight abutment, which allows an essentially spherical shape to be moulded. A fitting stud or bolt may be placed in each of the holes 35, 45 for precisely adjusting the position of the first and second mould portions 30 and 40 with respect to each other. Moreover, during the moulding process the fitting studs or bolts firmly press together the first mould portion 30 and the second mould portion 40 and hold them in tight abutment.

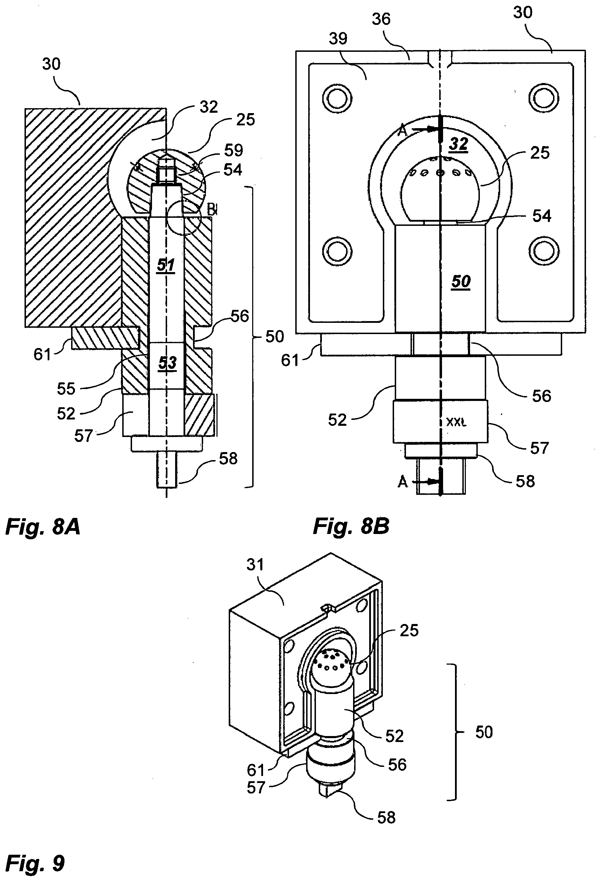

Referring now to Fig 8A and Fig 8B, a core positioning means 50 is adapted for being inserted into the semi-spherical recesses 33 and 43 in the first and second mould portions 30 and 40. The core positioning means 50 is essentially cylindrical and comprises a cylindrical bolt 51. At one end the bolt 51 features a tapered bolt neck 54 which is adapted for being inserted into the matching socket of the core element 25. At the end of the tapered bolt neck 54 a threaded end section 59 is provided for being screwed into a corresponding internal threading provided in the core element 25. Around a central part of the bolt 51, an external threading 53 is provided. At the other end, the bolt 51 is provided with a handle or dial 58. The core positioning means further comprises a sleeve 52 which surrounds the bolt 51, but leaves the bolt neck 54 free. The external diameter of the sleeve substantially corresponds with the internal diameter of the semi-cylindrical recesses 33 and 43 in the first and second mould portions 30 and 40. The internal diameter of the sleeve essentially corresponds to the external diameter of the bolt 51. A groove 56 is foreseen around the circumference of the sleeve 52. The groove 56 serves to fix the position of the sleeve with respect to a holding plate 61, which is attached to a sidewall of the first mould portion. The length of the sleeve 52 is selected such that is leaves a section of the bolt adjacent the handle or dial 58 free. This allows a position-setting washer 57 to be fitted to the free section of the bolt 51. The sleeve 52 further comprises an internal threading 55 adapted for engaging with the external threading 53 of the bolt 51. When the bolt 51 is inserted into the sleeve 52, the internal threading 55 engages with the external threading 53 of the bolt 51. By rotating the handle or dial 58, the position of the bolt neck 54 with respect to the sleeve 52 can be adjusted.

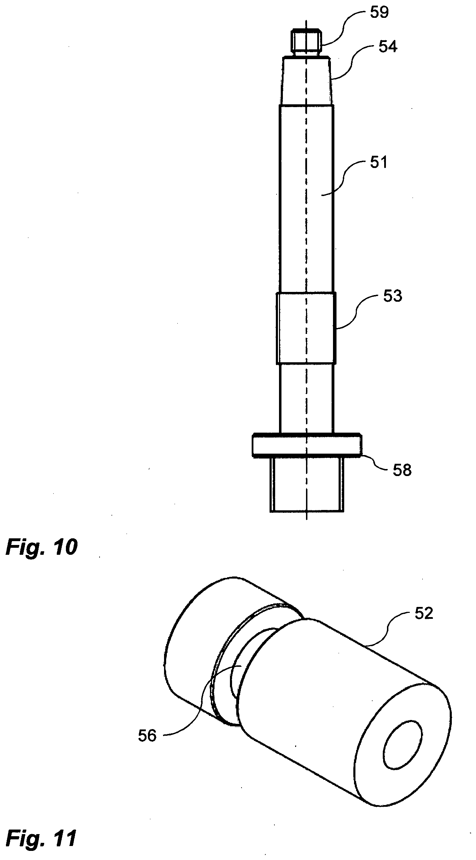

Fig 9 illustrates the first mould portion 30 with a holding plate 61, into which the core positioning means 50 has been inserted. A core element 25 is shown attached to the bolt neck. A C-shaped or slotted position-setting washer 57 is fitted to the free section of the bolt 51, shown in Fig 10. By rotating the handle or dial 58, the position of the bolt neck can be adjusted with respect to the sleeve 52 to correspond to the thickness of the position-setting washer 57. The thickness of the position setting washer 57 thus allows setting the position of the bolt neck 54, see Fig 10, and therefore also the position of the core element 25 within semi-spherical recess 32 of the first mould portion 30.

The cylindrical bolt 51 is shown in Fig 10. At one end the bolt 51 features a tapered bolt neck 54 which is adapted for being inserted into the matching socket of the core element 25. At the end of the tapered bolt neck 54 a threaded end section 59 is provided for being screwed into a corresponding internal threading provided in the core element 25. Around a central part of the bolt 51, an external threading 53 is provided which is adapted for engaging with the internal threading 55 of the sleeve 52. At the end of the bolt 51 opposite the bolt neck 54, a handle or dial 58 is provided.

As illustrated in Fig 11 a groove 56 is foreseen around the circumference of the sleeve 52. The groove 56 serves to fix the position of the sleeve with respect to a holding plate 61, which is attached to a sidewall of the first mould portion. The external diameter of the sleeve substantially corresponds with the diameter of the semi-cylindrical recesses and in the first and second mould portions. The internal diameter of the sleeve 52 essentially corresponds to the external diameter of the bolt 51. Thereby the groove 56 of the sleeve 52 engages with the holding plate, which secures the sleeve 52 against a translational movement in the axial direction. The length of the sleeve 52 is selected such that it leaves a section of the bolt adjacent the handle or dial 58 free. The sleeve 52 further comprises an internal threading 55 adapted for engaging with the external threading 53 of the bolt 51.

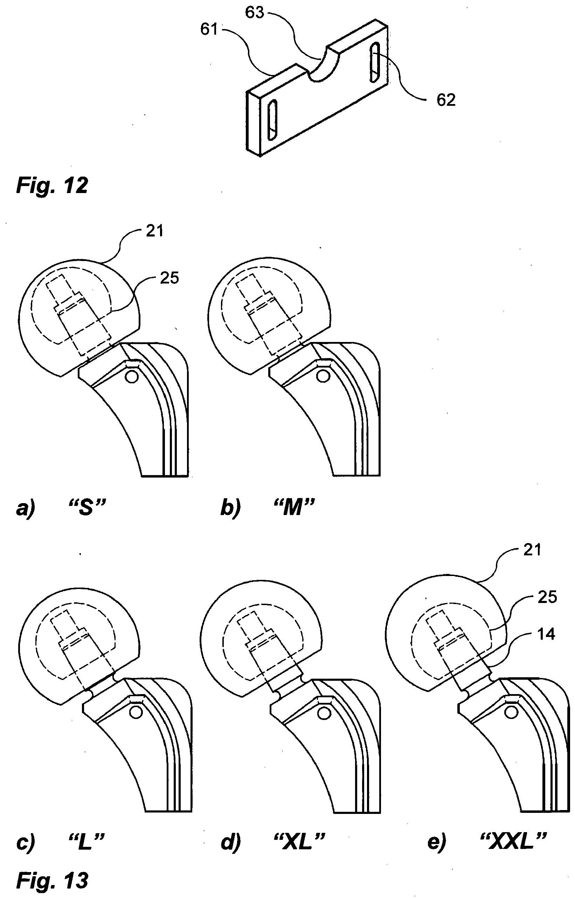

Fig 12 is an isometric representation of the holding plate 61. As described above with reference to Fig 8A and Fig 8B, the holding plate 61 is mounted to a sidewall 34 of the first mould portion 30 by means of fitting studs or bolts passing through long holes 62 provided in the holding plate 61. The fitting studs or bolts are attached to corresponding bore holes 38 provided in the sidewall 34 of the first mould portion 30. In order to hold the groove 56 in a predetermined position, the holding plate 61 is provided with a semi-circular recess 63, which substantially corresponds in diameter to the diameter of the groove 56 in the sleeve 52. Additionally, the thickness of the holding plate 61 is chosen to essentially match the width of the groove 56 in the axial direction.

In order to allow different positions to be set, a number of washers 57 of different thicknesses may be provided. It may be advantageous to use various predetermined washer thicknesses in order to produce the different sizes corresponding to the values determined in Table 1 and 2. The distance-setting washer 57 is attached to the distal end of the cylindrical bolt 51 and is locked into place by a dial 58 which from the second end of the cylindrical bolt 51.

As shown in Fig. 6 and 7, both the first mould 30 and the second mould 40 each comprise a semi-cylindrical channel 33, 43 extending from the central section of the mould along the intersecting plane to a proximal side wall 34, 44. In use, when first mould 30 and the second mould 40 face each other and are placed into abutment, these semi-cylindrical concave channels 33, 43 are aligned with each other, which allows a core positioning means 50 to be placed in the resulting cylindrical channel. The diameter of the sleeve 52 and the cylindrical channel 33, 43 are chosen such that in use they result in a tight fit, in order to prevent any liquid coating material from leaking.

At the proximal end of the first mould 30, a holding plate 61 is provided for supporting the part of the sleeve 52 that extends beyond the edge of the first mould 30.

It is understood by those skilled in the art that other distance setting means may be used without departing from the present invention. It may, e.g. be preferred to use core positioning means of different predetermined lengths instead of washers of different thickness. The effect, however, remains the same: the axial position of the core positioning means neck 54, and thereby also the position of the core element 25 within the moulds can be precisely determined prior to moulding.

### The manufacturing

The device comprising the first and second mould portions 30 and 40 is used for casting an outer coating 21 around the core element 25. The core element 25 is preferably chosen to be titanium of grade 2, which is destined for medical purposes. The material for casting the outer coating 21 is preferably chosen from biocompatible material, such as poly methyl methacrylate (PMMA), which is suitable for medical purposes. The material chosen for outer coating 21 may further comprise one or more pharmaceutically active substances, such as an antibiotic or antimycotic, which may be released from the material over time.

The device used for casting comprises a first mould portion 30 and a second mould portion 40. It is advantageous to choose e.g. polytetrafluoroethylene (PTFE), also known as Teflon^{®}, polyoxymethylene (POM-C) otherwise known as acetal copolymer, or another suitable material for the first mould portion 30 and the second mould portion 40. Of course, it is understood that other suitable materials might alternatively be used.

The core element 25 is attached to a core positioning means 50 with a conically-shaped neck 54. This conically shaped neck 54 has exactly the same angle as the implant neck 14 used in hip implants. The neck 54 further comprises a threaded end section 59 with a thread to which the core element 25 can be tightly screwed.

Since the core element 25 is always arranged at the same position on the conically shaped neck 54, the conically shaped neck 54 can translatingly be moved in the forward or backward direction by rotating the handle or dial 58. This allows adjusting the respective dimensions for *y* and *z* (see Fig 13 a to e) in order to manufacture the different sizes ranging from "S" to "XXL" per Table 2.

For manufacturing, the coating material is cast into the first mould portion 30 and the second mould portion 40. The two mould portions 30 and 40 are then joined. Advantageously bolts or studs (not shown in the drawings) are used for tightening and pressing the two mould portions 30 and 40 tightly against each other.

The fine thread 53 in the bolt 51 is preferably self-locking, so that the bolt 51 cannot dislocate due to the pressure of the mould portions or any exothermal solidification process of the coating material. This ensures compliance with the respective sizes. For easier handling, positioning devices 57, e.g. in the form of C-shaped or slotted washers 57 of different thicknesses may be provided and may advantageously be identified with e.g.: S, M, L, XL und XXL. These positioning washers 57 are form-fittingly inserted at the end between the sleeve 52 and the bolt 51.

After expiry of the dwell period the first mould portion 30 and the second mould portion 40 are separated. The finished prosthesis head 20 can then be removed from the mould portions 30 and 40. It may be advantageous to release any tension between the conical neck 54 and the core element 25 prior to removing the finished prosthesis head 20 by rotating the handle 58 on the threaded end section 59 of the cylindrical bolt 51, in order to separate of the PMMA coated core element 25 from the conical neck 54.

### The use in treatment

It may often not be advisable, or even possible, to remove the entire implant, when the infection is restricted to the acetabulum or when the removal of the implant out of the femur is live threatening.

In accordance with a further aspect of the present invention it is therefore proposed to leave the main body of the implant in place in the femur in these cases and to merely replace the original implant head by a prosthesis head which has been obtained in accordance with the present invention. At the same time, the acetabular cup is removed from the pelvic bone.

Once the infection has healed, the prosthesis head may be removed and be replaced by a definitive implant head. At the same time, the hip joint socket is again placed into the pelvic bone.

While the technique presented herein has been described in relation to its preferred embodiments, it is to be understood that this description is for illustrative purposes only. It will be appreciated by the person of skill in the art that various modifications may be made to the above described embodiments without departing from the scope of the present invention. Accordingly, it is intended that the invention be limited only by the scope of the claims appended hereto.

**List of reference numbers**

| | | | |
|---|---|---|---|
| 10 | femoral prosthesis | 37 | groove |
| 11 | femoral implant body | 39 | recessed plane |
| 13 | proximal end | 40 | second mould portion |
| 14 | implant neck | 41 | second face |
| 15 | prosthesis head | 42 | semi-spherical recess |
| 16 | tapered socket | 43 | semi-cylindrical recess |
| 17 | cylindrical protrusion | 44 | adjacent sidewall |
| 18 | hip socket joint | 45 | hole |
| 19 | pelvic bone | 46 | rim |
| 20 | prosthesis head | 47 | groove |
| 21 | outer coating | 49 | recessed plane |
| 22 | cut-out | 50 | core positioning means |
| 25 | core element | 51 | cylindrical bolt |
| 26 | socket | 52 | sleeve |
| 27 | bore | 53 | external threading |
| 28 | threading | 54 | bolt neck |
| 29 | recess | 55 | internal threading |
| 30 | first mould portion | 56 | groove |
| 31 | first face | 57 | position-setting washer |
| 32 | semi-spherical recess | 58 | handle or dial |
| 33 | semi-cylindrical recess | 59 | threaded end section |
| 34 | adjacent sidewall | 61 | holding plate |
| 35 | hole | 62 | long hole |
| 36 | rim | 63 | semi-circular recess |

## Claims

1. A device for manufacturing a prosthesis head, the device comprising
a mould (30, 40), the mould comprising a first mould portion (30) and a second mould portion (40) and a substantially spherical cavity (32, 42), optionally in the form of a substantially spherical segment;
a core positioning means (50) having a longitudinal axis and comprising a first end (54), the first end (54) adapted for receiving a socket of a core element of a prosthesis head;
wherein the mould (30, 40) comprises means (33, 43) for receiving the core positioning means (50), so that the first end (54) of the core positioning means (50) rests within the substantially spherical mould cavity (32, 42), and
wherein the means (33, 43) further align the longitudinal axis of the core positioning means (50) with a radial direction with respect to the spherical cavity (32, 42), **characterized in that**
the means (33, 43) for aligning the longitudinal axis of the core positioning means (50) comprise
in the first mould portion (30), a first semi-cylindrical channel (33) extending from the first semi-spherical concave recess (32) to an adjacent side wall (34); and
in the second mould portion (40), a second semi-cylindrical channel (43) extending from the second semi-spherical concave recess (42) to an adjacent side wall (44).

2. The device of claim 1, wherein the core positioning means (50) is of substantially the same diameter as the semi-cylindrical channels (33, 43) of the first and second mould portions (30, 40), and wherein the core positioning means (50) comprises a neck portion at its proximal end (54) for attaching a core element.

3. The device of claim 2, wherein the core positioning means (50) further comprises a bolt (51), wherein the neck portion is provided at the proximal end (54) of the bolt (51).

4. The device of any one of claims 2 to 3, wherein the core positioning means (50) further comprises means for adjusting the position of its neck portion (54) to a predetermined position when placed into the substantially spherical cavity (32, 42).

5. The device of claim 4, wherein the means for adjusting the position of the neck portion (54) to a predetermined position comprise an outer threading at the distal end of the bolt (51).

6. A method of manufacturing a prosthesis head, the method comprising the steps of:
a) providing a core element (25);
b) providing a core positioning means (50) having a longitudinal axis and comprising a first end (54), the first end (54) adapted for receiving a socket of a core element of a prosthesis head;
c) providing a mould (30, 40), the mould comprising a substantially spherical cavity (32, 42), wherein the mould (30, 40) further comprises means (33, 43) for receiving the core positioning means (50);
d) attaching the core element (25) to the first core positioning means end (54);
e) introducing the core positioning means into the mould (30, 40), so that the first end (54) of the core positioning means (50) rests within the substantially spherical mould cavity (32, 42) and the longitudinal axis of the core positioning means (50) is aligned with a radial direction with respect to the substantially spherical cavity (32, 42); and
f) filling the remainder of the spherical cavity (32, 42) with a coating material for coating the core element (25),
**characterized in that** step c) further comprises
providing a first mould portion (30), the first mould portion comprising a first semi-spherical concave recess (32) and a first semi-cylindrical channel (33) extending from the first semi-spherical concave recess (32) of the mould along the intersecting plane to a first proximal side wall (34); and
providing a second mould portion (40), the second mould portion comprising a second semi-spherical concave recess (42) and a second semi-cylindrical channel (43) extending from the second semi-spherical concave recess (42) of the mould along the intersecting plane to a second proximal side wall (44).

7. The method of claim 6, wherein step b) further comprises
providing a core positioning means (50) of substantially the same diameter as the first and second semi-cylindrical concave recesses (33, 43) of the first and second mould portions (30, 40), the core positioning means (50) comprising a neck portion for attaching a core element (25) at the first end (54) of the core positioning means (50).

8. The method of any one of claims 6 and 7, wherein step d) further comprises
attaching the core element (25) to the neck portion of the first end (54) of core positioning means (50).

9. The method of any one of claims 6 to 8, wherein step f) further comprises
f1) providing a mouldable coating material;
f2) inserting the mouldable coating material into the first and second semi-spherical concave recesses (32, 42) of the first and second mould portions (30, 40);
f3) inserting the core element (25) into the mouldable material provided in the first semi-spherical concave recess (32) of the first mould portion (30), such that the attached core positioning means (50) is inserted into the first semi-cylindrical channel (33);
f4) placing the second mould portion (40) in abutment with the first mould portion (30) so that the semi-spherical concave recesses (32, 42) face each other and are aligned and placed in tight abutment such that the semi-cylindrical concave channels (33, 43) are also aligned with each other, whereby the core element (25) is inserted into the mouldable material provided in the second semi-spherical concave recess (42) of the second mould portion (40),
f5) allowing the mouldable coating material to cure;
f6) separating the first mould portion (30) from the second mould portion (40), and removing the core positioning means (50) from the coated core element (25), such that a substantially spherical prosthesis head (20) is obtained in the form of core element (25) coated with a moulded outer coating (21).

10. The method of claim 9, wherein the length of the core positioning means (50) is selected such that a desired axial displacement between the core element (25) and the outer coating (21) results.

11. The method of claim 10, further comprising adjusting the length of the core positioning means (50).

12. The method of claim 10, further comprising selecting a core positioning means (50) of desired length.

## Patentansprüche

1. Vorrichtung zum Herstellen eines Prothesenkopfs, wobei die Vorrichtung umfasst:
eine Form (30, 40), wobei die Form einen ersten Formenteil (30) und einen zweiten Formenteil (40) und eine im Wesentlichen kugelförmige Aussparung (32, 42) umfasst, wahlweise in Form eines im Wesentlichen kugelförmigen Segments;
ein Grundkörperpositionierungsmittel (50), das eine Längsachse aufweist und ein erstes Ende (54) umfasst, wobei das erste Ende (54) zum Aufnehmen einer Buchse eines Grundkörperelements eines Prothesenkopfs geeignet ist;
wobei die Form (30, 40) ein Mittel (33, 43) zum Aufnehmen des Grundkörperpositionierungsmittels (50) umfasst, so dass das erste Ende (54) des Grundkörperpositionierungsmittels (50) innerhalb der im Wesentlichen kugelförmigen Formaussparung (32, 43) ruht, und
wobei das Mittel (33, 43) ferner die Längsachse des Grundkörperpositionierungsmittels (50) in einer radialen Richtung bezüglich der kugelförmigen Aussparung (32, 42) ausrichtet, **dadurch gekennzeichnet, dass** das Mittel (33, 43) zum Ausrichten der Längsachse des Grundkörperpositionierungsmittels (50) umfasst:
im ersten Formenteil (30) eine erste halbzylinderförmige Auskehlung (33), die sich von der ersten halbkugelförmigen konkaven Ausnehmung (32) zu einer benachbarten Seitenwand (34) erstreckt; und
im zweiten Formenteil (40) eine zweite halbzylinderförmige Auskehlung (43), die sich von der zweiten halbkugelförmigen konkaven Ausnehmung (42) zu einer benachbarten Seitenwand (44) erstreckt.

2. Vorrichtung nach Anspruch 1, wobei das Grundkörperpositionierungsmittel (50) im Wesentlichen den gleichen Durchmesser wie die halbzylinderförmigen Auskehlungen (33, 43) des ersten und des zweiten Formenteils (30, 40) aufweist und wobei das Grundkörperpositionierungsmittel (50) an seinem proximalen Ende (54) einen Halsabschnitt zum Anbringen eines Grundkörperelements umfasst.

3. Vorrichtung nach Anspruch 2, wobei das Grundkörperpositionierungsmittel (50) ferner einen Dorn (51) umfasst, wobei der Halsabschnitt am proximalen Ende (54) des Doms (51) bereitgestellt ist.

4. Vorrichtung nach einem der Ansprüche 2 bis 3, wobei das Grundkörperpositionierungsmittel (50) ferner ein Mittel zum Anpassen der Position seines Halbabschnitts (54) auf eine vorgegebene Position, wenn dieser sich in der im Wesentlichen kugelförmigen Aussparung (32, 42) befindet, umfasst.

5. Vorrichtung nach Anspruch 4, wobei das Mittel zum Anpassen der Position des Halsabschnitts (54) auf eine vorgegebene Position am distalen Ende des Dorns (51) ein Außengewinde umfasst.

6. Verfahren zum Herstellen eines Prothesenkopfs, wobei das Verfahren die Schritte umfasst:
a) Bereitstellen eines Grundkörperelements (25);
b) Bereitstellen eines Grundkörperpositionierungsmittels (50), das eine Längsachse aufweist und ein erstes Ende (54) umfasst, wobei das erste Ende (54) zum Aufnehmen einer Buchse eines Grundkörperelements eines Prothesenkopfs geeignet ist;
c) Bereitstellen einer Form (30, 40), wobei die Form eine im Wesentlichen kugelförmige Aussparung (32, 42) umfasst, wobei die Form (30, 40) ferner ein Mittel (33, 43) zum Aufnehmen des Grundkörperpositionierungsmittels (50) umfasst;
d) Anbringen des Grundkörperelements (25) am ersten Ende (54) des Grundkörperpositionierungsmittels;
e) Einbringen des Grundkörperpositionierungsmittels in die Form (30, 40), so dass das erste Ende (54) des Grundkörperpositionierungsmittels (50) innerhalb der im Wesentlichen kugelförmigen Formaussparung (32, 42) ruht und die Längsachse des Grundkörperpositionierungsmittels (50) in eine radiale Richtung bezüglich der im Wesentlichen kugelförmigen Aussparung (32, 42) ausgerichtet ist; und
f) Auffüllen der verbleibenden kugelförmigen Aussparung (32, 42) mit einem Umhüllungsmaterial zum Umhüllen des Grundkörperelements (25),
**dadurch gekennzeichnet, dass** Schritt c) ferner umfasst:
Bereitstellen eines ersten Formenteils (30), wobei der erste Formenteil eine erste halbkugelförmige konkave Ausnehmung (32) und eine erste halbzylinderförmige Auskehlung (33) umfasst, die sich von der ersten halbkugelförmigen konkaven Ausnehmung (32) der Form entlang einer Schnittebene zu einer ersten proximalen Seitenwand (34) erstreckt; und
Bereitstellen eines zweiten Formenteils (40), wobei der zweite Formenteil eine zweite halbkugelförmige konkave Ausnehmung (42) und eine zweite halbzylinderförmige Auskehlung (43) umfasst, die sich von der zweiten halbkugelförmigen konkaven Ausnehmung (42) der Form entlang der Schnittebene zu einer zweiten proximalen Seitenwand (44) erstreckt.

7. Verfahren nach Anspruch 6, wobei Schritt b) ferner umfasst:
Bereitstellen eines Grundkörperpositionierungsmittels (50) mit im Wesentlichen dem gleichen Durchmesser wie die erste und die zweite halbzylinderförmige konkave Ausnehmung (33, 43) des ersten und des zweiten Formenteils (30, 40), wobei das Grundkörperpositionierungsmittel (50) einen Halsabschnitt zum Anbringen eines Grundkörperelements (25) am ersten Ende (54) des Grundkörperpositionierungsmittels (50) umfasst.

8. Verfahren nach einem der Ansprüche 6 bis 7, Schritt d) ferner umfasst:
Anbringen des Grundkörperelements (25) am Halsabschnitt des ersten Endes (54) des Grundkörperpositionierungsmittels (50).

9. Verfahren nach einem der Ansprüche 6 bis 8, Schritt f) ferner umfasst:
f1) Bereitstellen eines formbaren Umhüllungsmaterials;
f2) Einbringen des formbaren Umhüllungsmaterials in die erste und die zweite halbkugelförmige konkave Ausnehmung (32, 42) des ersten und des zweiten Formenteils (30, 40);
f3) Einbringen des Grundkörperelements (25) in das in der ersten halbkugelförmigen konkaven Ausnehmung (32) des ersten Formenteils (30) befindliche formbare Material, so dass das angebrachte Grundkörperpositionierungsmittel (50) in die erste halbzylinderförmige Auskehlung (33) eingebracht wird;
f4) Inauflagebringen des zweiten Formenteils (40) mit dem ersten Formenteil (30), so dass die halbkugelförmigen konkaven Ausnehmungen (32, 42) einander zugewandt und ausgerichtet sind und in dichte Auflage gebracht sind, so dass die halbzylinderförmigen konkaven Auskehlungen (33, 43) ebenfalls miteinander ausgerichtet sind, wobei das Grundkörperelement (25) in das in der zweiten halbkugelförmigen konkaven Ausnehmung (42) des zweiten Formenteils (40) befindliche formbare Material eingebracht wird,
f5) Aushärtenlassen des formbaren Umhüllungsmaterials;
f6) Trennen des ersten Formenteils (30) vom zweiten Formenteil (40) und Abnehmen des Grundkörperpositionierungsmittels (50) von dem umhüllten Grundkörperelement (25), so dass ein im Wesentlichen kugelförmiger Prothesenkopf (20) in Form eines mit einer geformten äußeren Umhüllung (21) umhüllten Grundkörperelements (25) erhalten wird.

10. Verfahren nach Anspruch 9, wobei die Länge des Grundkörperpositionierungsmittels (50) so ausgewählt ist, dass eine gewünschte axiale Versetzung zwischen dem Grundkörperelement (25) und der äußeren Umhüllung (21) erhalten wird.

11. Verfahren nach Anspruch 10, das ferner das Anpassen der Länge des Grundkörperpositionierungsmittels (50) umfasst.

12. Verfahren nach Anspruch 10, das ferner das Auswählen eines Grundkörperpositionierungsmittels (50) mit einer gewünschten Länge umfasst.

## Revendications

1. Dispositif pour fabriquer une tête de prothèse, le dispositif comprenant :
un moule (30, 40), le moule comprenant la première partie de moule (30) et une seconde partie de moule (40) et une cavité sensiblement sphérique (32, 42), facultativement sous la forme d'un segment sensiblement sphérique ;
un moyen de positionnement de noyau (50) ayant un axe longitudinal et comprenant une première extrémité (54), la première extrémité (54) étant adaptée pour recevoir une douille d'un élément de noyau d'une tête de prothèse ;
dans lequel le moule (30, 40) comprend des moyens (33, 43) pour recevoir le moyen de positionnement de noyau (50), de sorte que la première extrémité (54) du moyen de positionnement de noyau (50) s'appuie à l'intérieur de la cavité de moule sensiblement sphérique (32, 42), et
dans lequel les moyens (33, 43) alignent l'axe longitudinal du moyen de positionnement de noyau (50) avec une direction radiale par rapport à la cavité sphérique (32, 42), **caractérisé en ce que** :
les moyens (33, 43) pour aligner l'axe longitudinal du moyen de positionnement de noyau (50) comprennent :
dans la première partie de moule (30), un premier canal semi-cylindrique (33) s'étendant à partir du premier évidement concave semi-sphérique (32) à une paroi latérale (34) adjacente ; et
dans la seconde partie de moule (40), un second canal semi-cylindrique (43) s'étendant à partir du second évidement concave semi-sphérique (42) jusqu'à une paroi latérale (44) adjacente.

2. Dispositif selon la revendication 1, dans lequel le moyen de positionnement de noyau (50) a sensiblement le même diamètre que les canaux semi-sphériques (33, 43) des première et seconde parties de moule (30, 40), et dans lequel le moyen de positionnement de noyau (50) comprend une partie de col au niveau de son extrémité proximale (54) pour fixer un élément de noyau.

3. Dispositif selon la revendication 2, dans lequel le moyen de positionnement de noyau (50) comprend en outre un boulon (51), dans lequel la partie de col est prévue au niveau de l'extrémité proximale (54) du boulon (51).

4. Dispositif selon l'une quelconque des revendications 2 à 3, dans lequel le moyen de positionnement de noyau (50) comprend en outre des moyens pour ajuster la position de sa partie de col (54) dans une position prédéterminée lorsqu'il est placé dans la cavité sensiblement sphérique (32, 42).

5. Dispositif selon la revendication 4, dans lequel les moyens pour ajuster la position de la partie de col (54) dans une position prédéterminée comprennent un filetage externe au niveau de l'extrémité distale du boulon (51).

6. Procédé pour fabriquer une tête de prothèse, le procédé comprenant les étapes suivantes :
a) prévoir un élément de noyau (25) ;
b) prévoir un moyen de positionnement de noyau (50) ayant un axe longitudinal et comprenant une première extrémité (54), la première extrémité (54) étant adaptée pour recevoir une douille d'un élément de noyau d'une tête de prothèse ;
c) prévoir un moule (30, 40), le moule comprenant une cavité sensiblement sphérique (32, 42), dans lequel le moule (30, 40) comprend en outre des moyens (33, 43) pour recevoir le moyen de positionnement de noyau (50) ;
d) fixer l'élément de noyau (25) sur la première extrémité (54) du moyen de positionnement de noyau ;
e) introduire le moyen de positionnement de noyau dans le moule (30, 40), de sorte que la première extrémité (54) du moyen de positionnement de noyau (50) s'appuie à l'intérieur de la cavité de moule sensiblement sphérique (32, 42) et l'axe longitudinal du moyen de positionnement de noyau (50) est aligné avec une direction radiale par rapport à la cavité sensiblement sphérique (32, 42) ; et
f) remplir le reste de la cavité sphérique (32, 42) avec un matériau de revêtement pour recouvrir l'élément de noyau (25),
**caractérisé en ce que** l'étape c) comprend en outre les étapes suivantes :
prévoir une première partie de moule (30), la première partie de moule comprenant un premier évidement (32) concave semi-sphérique et un premier canal (33) semi-cylindrique s'étendant à partir du premier évidement (32) concave semi-sphérique du moule le long du plan d'intersection jusqu'à une première paroi latérale (34) proximale ; et
prévoir une seconde partie de moule (40), la seconde partie de moule comprenant un second évidement (42) concave semi-sphérique et un second canal (43) semi-cylindrique s'étendant à partir du second évidement (42) concave semi-sphérique du moule le long du plan d'intersection jusqu'à une seconde paroi latérale (44) proximale.

7. Procédé selon la revendication 6, dans lequel l'étape b) comprend en outre l'étape suivante :
prévoir un moyen de positionnement de noyau (50) ayant sensiblement le même diamètre que les premier et second évidements (33, 43) concaves semi-cylindriques des première et seconde parties de moule (30, 40), le moyen de positionnement de noyau (50) comprenant une partie de col pour fixer un élément de noyau (25) au niveau de la première extrémité (54) du moyen de positionnement de noyau (50).

8. Procédé selon l'une quelconque des revendications 6 et 7, dans lequel l'étape d) comprend en outre l'étape suivante :
fixer l'élément de noyau (25) à la partie de col de la première extrémité (54) du moyen de positionnement de noyau (50).

9. Procédé selon l'une quelconque des revendications 6 à 8, dans lequel l'étape f) comprend en outre les étapes suivantes :
f1) prévoir un matériau de revêtement pouvant être moulé ;
f2) insérer le matériau de revêtement pouvant être moulé dans les premier et second évidements (32, 42) concaves semi-sphériques des première et seconde parties de moule (30, 40) ;
f3) insérer l'élément de noyau (25) dans le matériau pouvant être moulé prévu dans le premier évidement (32) concave semi-sphérique de la première partie de moule (30), de sorte que le moyen de positionnement de noyau (50) fixé est inséré dans le premier canal (33) semi-cylindrique ;
f4) placer la seconde partie de moule (40) en butée avec la première partie de moule (30) de sorte que les évidements (32, 42) concaves semi-sphériques se font face et sont alignés et placés en butée serrée de sorte que les canaux (33, 43) concaves semi-cylindriques sont également alignés entre eux, moyennant quoi l'élément de noyau (25) est inséré dans le matériau pouvant être moulé prévu dans le second évidement (42) concave semi-sphérique de la seconde partie de moule (40),
f5) permettre au matériau de revêtement pouvant être moulé de durcir ;
f6) séparer la première partie de moule (30) de la seconde partie de moule (40) et retirer le moyen de positionnement de noyau (50) de l'élément de noyau (25) recouvert, de sorte qu'une tête de prothèse (20) sensiblement sphérique est obtenue sous la forme de l'élément de noyau (25) recouvert avec un revêtement externe (21) moulé.

10. Procédé selon la revendication 9, dans lequel la longueur du moyen de positionnement de noyau (50) est sélectionnée de sorte qu'il en résulte un déplacement axial souhaité entre l'élément de noyau (25) et le revêtement externe (21).

11. Procédé selon la revendication 10, comprenant en outre l'ajustement de la longueur du moyen de positionnement de noyau (50).

12. Procédé selon la revendication 10, comprenant en outre la sélection d'un moyen de positionnement de noyau (50) de longueur souhaitée.
